(19) Europäisches Patentamt
European Patent Office
Office européen des brevets

(11) Publication number: **0 215 200 B1**

(12) **EUROPEAN PATENT SPECIFICATION**

(45) Date of publication of patent specification: **09.09.92**

(51) Int. Cl.$^5$: **A61K 31/505**

(21) Application number: **86108295.6**

(22) Date of filing: **18.06.86**

(54) **Use of sulfanilamido quinoxalines in the treatment of neoplastic diseases.**

(30) Priority: **24.06.85 US 748070**

(43) Date of publication of application:
**25.03.87 Bulletin 87/13**

(45) Publication of the grant of the patent:
**09.09.92 Bulletin 92/37**

(84) Designated Contracting States:
**CH DE FR GB IT LI NL**

(56) References cited:

**DIALOG INFORMATION SERVICES, file 159, no. 9582, MENDELEEV IM et al.: "Isolated lymphogranulomatosis of the stomach and intestine", & KLIN MED (MOSK); 45(4): 64-71 1967**

**ACTO CRYSTELLOGR. SECT. C: CRYST. STRUCT. COMMUN., vol. C41, no. 7, 1985, pages 1116-1118, International Union of Crystallography; H.M. DEUTSCH et al.: "Structure of the acetonitrille solvate of the antitumor agent 4-amino-N-(5-chloro-2-quinoxalinyl)benzene-sulfonamide, C14H11ClN4O2S1CH3CN"**

(73) Proprietor: **MERCK & CO. INC.**
**126, East Lincoln Avenue P.O. Box 2000**
**Rahway New Jersey 07065-0900(US)**

(72) Inventor: **Tolman, Richard L.**
**29 Upper Warren Way**
**Warren, N.J. 07060(US)**

(74) Representative: **Blum, Rudolf Emil Ernst et al**
**c/o E. Blum & Co Patentanwälte Vorderberg 11**
**CH-8044 Zürich(CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 215 200 B1

**AN. REAL. ACAD. FARM., vol. 46, no. 2, 1980, pages 153-166; A. DOADRIO et al.: "Structure-antitumour activity relationship for the Pt2 + ,Pd3 + ,Pd4 + ,Os4 + complexes and complex salts with sulphonamide derivatives"**

**INDEX PHARMACORUM, 1980, page 430, no. 16l6.8, Georg Thieme Verlag, Stuttgart, DE; W. KOLL: "Sulfa-chinoxalin"**

**BIOCHEMICAL PHARMACOLOGY, vol. 37, no. 23, 1988, pages 4557-4564, Pergamon Press, GB; R.F. BRANDA et al.: "Cellular pharmacology of chloroquinoxaline sulfonamide and a related compound in murine B16 melanoma cells"**

**F.W.Wiselogle, Ed."A survey of antimalarial drugs, 1941-1945", J.W.Edwards, Ann Arbor, Mich 1946 (p. 1467-1470)**

## Description

### BACKGROUND OF THE INVENTION

The present invention concerns the use of sulfanilamido quinoxalines as active ingredient for the preparation of pharmaceutical compositions for the treatment of a neoplastic disease. The sulfanilamido quinoxalines which are used for the preparation of said pharmaceutical compositions comprise such sulfonilamido quinoxalines which are already described in the prior art as well as also corresponding new compounds. The present invention furthermore concerns several new sulfanilamido quinoxalines.

### DESCRIPTION OF THE PRIOR ART

Pertinent to the background of this invention is the compound 2-(4-aminobenzenesulfonamido)-quinoxaline, also known as sulfaquinoxaline, which has been used as a coccidiostat in the prophylactic treatment of chickens and other fowl to prevent the onset of coccidiosis disease. Also in the U.S. patent no. 2 404 199, there is described the 2-(4-aminobenzenesulfonamido)-quinoxaline of formula A

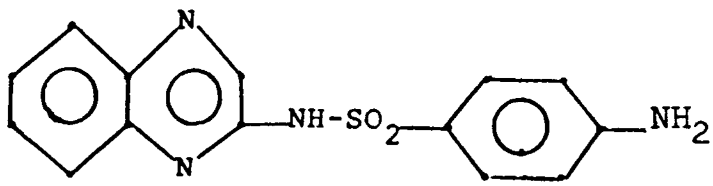

A

and there is stated therein that said compound has bactericidal properties. Said active ingredient of formula A is prepared according to said patent by reacting 2-aminoquinoxaline with the 4-aminobenzenesulfochloride in which the amino group is protected by acylation, yielding the corresponding intermediate product having an acylated amino group which is split off by hydrolysis yielding the compound of formula A. An improved process for the preparation of said compound of formula A is described in the U.S. patent 2 675 379, according to which said compound is prepared by reacting a 2-haloquinoxaline with sulfanylamide in an inert high boiling solvent and in the presence of a solid acid-binding agent.

In an English language abstract of the Russian language publication, Dialog Information Services, file 159, no. 9582, Mendeleev Im et al.: "Isolated lymphogranulomatosis of the stomach and intestine" & Klin Med (Mos); 45(4(: 64-71 1967, a clinical report is made about different methods of chemotherapy, x-ray therapy and surgical treatment of patients suffering from Hodgkin's disease. One patient with jejunal Hodgkin's disease was treated with surgery, followed by a treatment with xylyl-bis(2-chloroethyl)amine, which resulted in a remission and after a recurrence there followed a treatment with at least 2,2 g embazin, a further surgical treatment and radiotherapy, and finally the patient was released in good condition. Nothing is stated about the further development of the health condition of said single person who was submitted to the treatments. As radiotherapy, however, is a common method of treating Hodgkin's disease, from said publication there cannot be seen whether one of the three therapeutic methods, embazin, surgery or radiotherapy, or a combination thereof, resulted in the successful treatment.

In the publication Index Pharmacorum, 1980, page 430, no.1616.8, the compound of formula A, i.e. embazin, is described as a sulphonamide having antibacterial activity. The antitumor activity of complexes of several sulphonamide derivatives with ions of several noble metals, i.e. the ions of platinum, palladium and osmium, was tested in the publication AN. Real. Acad. Farm. vol. 46, no. 2, 1980, pages 153-166. Among the tested complexes with the noble metals, also the complex of the compound having the structure A stated before was tested.

Also pertinent to the present invention is an article published by F.J. Wolf et al., Substituted Sulfaquinoxaline III Extension of the Glyoxalate Synthesis of 2-Aminoquinoxaline, J. Am. Chem. Soc. 71, 6-10 (1947) which describes the synthesis of a number of similarly substituted sulfanylaminoquinoxalines and some of the compounds were intended to be used in antimalarial testing (no results are given). According to said general method of synthesis first 2-aminoquinoxalines are prepared which are substituted in the benzene ring of the quinoxaline structure with alkyl-, alkoxy-, nitro-, amino-carboxylic acid groups or corresponding ester groups or sulfonic acid or sulfonamide groups or with a halogen atom, and said substituted 2-aminoquinoxalines are then reacted with a p-acetylaminobenzenesulfonyl chloride to produce the 2-$N^4$-acetyl-sulfanilamide quinoxalines. The hydrolyzation of said acetyl protecting group yields derivatives of the compounds of formula A having the above mentioned substituent in the benzene ring of the

quinoxaline structure.

In the publication F.W. Wiselogle, Ed., "A Survey of Antimalarial Drugs, 1941-1945, J.W. Edwards, Ann Arbor, Mich., 1946, pages 1467-1470, there are described several sulfanilamido-quinoxalines which had been tested for their antimalarial activity. Among the tested compounds there is to be found the 2-sulfanilamido-5-chloro-quinoxaline.

It was now quite unexpectedly found out that several sulfanylaminoquinoxalines, which are structurally related to the compounds of formula A, have quite generally a neoplasm inhibiting activity. Said compounds show in vitro tests an inhibiting activity onto the growth of several human tumor colony forming cells. The corresponding sulfanylamino-quinoxalines comprise compounds which were already described in the prior art (see e.g. the above stated publication of Wolf et al.) as well as also new derivatives of related structures to the one described in the prior art.

The sulfanylaminoquinoxalines are used according to the present invention as active ingredients of pharmaceutical compositions for treating neoplastic diseases.

## DESCRIPTION OF THE INVENTION

One object of the present invention accordingly is the use of a compound of formula I

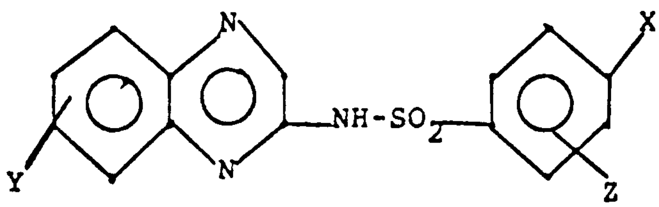

I

wherein

Y is a substituent attached to any of the unsubstituted carbons on the quinoxaline ring which substituent is selected from $NH_2$, $NO_2$, $OCH_3$, hydroxy, hydrogen, methyl, fluoro, chloro, bromo or carboxy;

Z is hydrogen or halo; and

X is selected from $NO_2$, acetylated amino groups and groups having the formula:

$NH-(CH_2)_n-COOH$ or $NHCH_2SO_3H$

wherein

n is an integer of 1 - 6 and wherein furthermore in those compounds of formula I wherein at least one of the radicals Y and Z has another meaning than the hydrogen atom, the radical X can furthermore have the meaning of an amino group of formula $NH_2$,

as active ingredient for the preparation of pharmaceutical compositions for the treatment of a neoplastic disease.

According to a preferred embodiment of the present invention there is used as compound of formula I for the preparation of the corresponding pharmaceutical composition the 2-sulfanilamido-5-chloroquinoxaline

A further object of the present invention are those compounds of formula I which are new compounds and in which accordingly corresponds to the following formula Ia

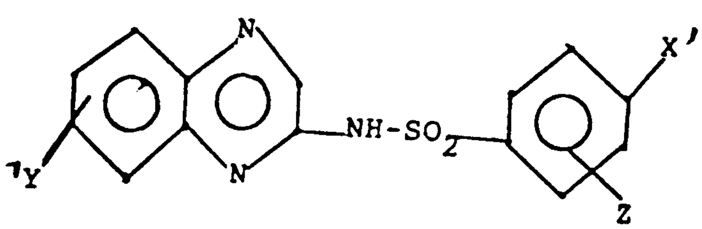

I a

wherein

Y' is a substituent attached to any of the unsubstituted carbons on the quinoxaline ring which substituent is selected from $NH_2$, $NO_2$, $OCH_3$, hydroxy, hydrogen, methyl, fluoro, chloro, bromo

or carboxy

Z is hydrogen or halo; and

X' is selected from $NO_2$, $NH_2$, acetylated amino group and groups having the formula

$NH-(CH_2)_n-COOH$ or $NHCH_2SO_3H$

wherein

n is an integer of 1 - 6

with the provision that X' must not be a free or acetylated amino group if Z is hydrogen and furthermore Y' is $NH_2$, $NO_2$, $OCH_3$, hydrogen, methyl, fluoro, chloro, bromo or carboxy.

Preparation of Active Compounds

The compounds active as antineoplastic agents of formula I hereinabove are prepared in accordance with the procedure set forth in the article by F.J. Wolf et al., Substituted Sulfaquinoxaline III Extension of the Glyoxalate Synthesis of 2-Aminoquinoxaline, J. Am. Chem. Soc. 71, 6-10 (1947). The process described therein comprises treatment of an appropriately substituted 2-aminoquinoxaline with a p-nitro- or p-acetylaminobenzenesulfonyl chloride in dry pyridine and if desired to produce the corresponding 2-(4-nitrobenzenesulfonylamido)quinoxalineor the 2-$N^4$-acetylsulfanilamidoquinoxaline and if desired hydrolyzing the N-acetyl compounds to produce the corresponding 2-sulfanilamidoquinoxaline as illustrative herein below:

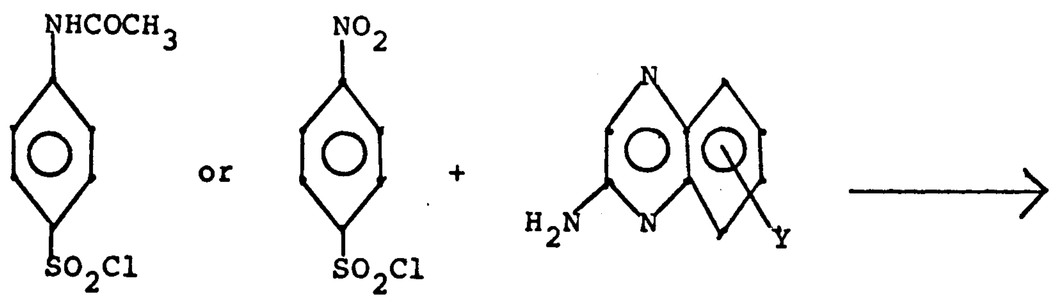

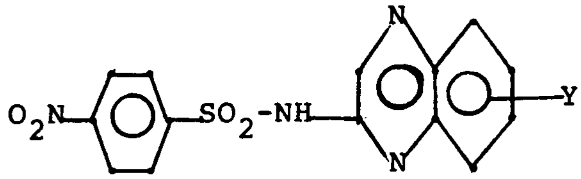

or

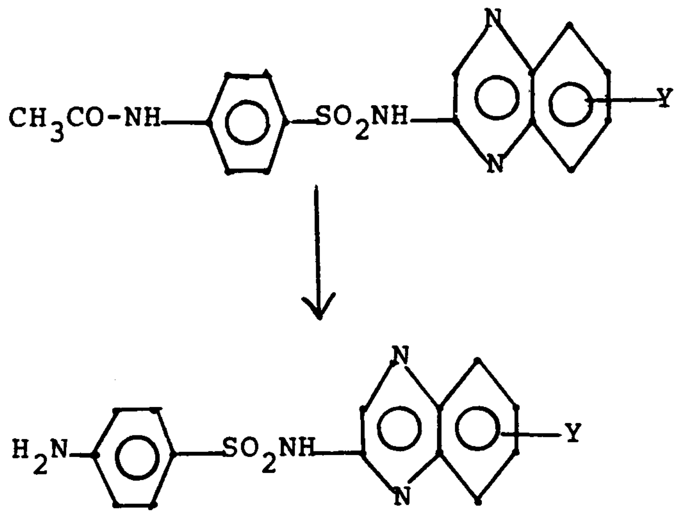

Compounds prepared in this manner are:

| | | MP |
|---|---|---|
| 2-(4-nitrobenzenesulfonylamido)-quinoxaline | | |
| 2-sulfanilamido-quinoxaline | | |
| 2-sulfanilamido-6 (or 7)-methyl quinoxaline | a | 215-16 ° C |
| 2-sulfanilamido-6 (or 7)-methyl quinoxaline | b | 255-56 ° C |
| 2-sulfanilamido-6 (or 7)-chloro quinoxaline | a | 248-49 ° C |
| 2-sulfanilamido-6 (or 7)-chloro quinoxaline | b | 238-39 ° C |
| 2-sulfanilamido-6 (or 7)-bromo quinoxaline | a | 244-46 ° C |
| 2-sulfanilamido-6 (or 7)-bromo quinoxaline | b | 239-40 ° C |
| 2-sulfanilamido-6 (or 7)-nitro quinoxaline | a | 220-21 ° C |
| 2-sulfanilamido-6 (or 7)-nitro quinoxaline | b | 224-26 ° C |
| 2-sulfanilamido-6 (or 7)-amino quinoxaline | a | 275-76 ° C |
| 2-sulfanilamido-6 (or 7)-methoxy quinoxaline | a | 239-40 ° C |
| 2-sulfanilamido-6 (or 7)-methoxy quinoxaline | b | 235-37 ° C |
| 2-sulfanilamido-6 (or 7)-carboxy quinoxaline | b | 223 ° C |
| 2-sulfanilamido-5 (or 8)-methyl quinoxaline | a | 205-06 ° C |
| 2-sulfanilamido-5 (or 8)-methyl quinoxaline | b | 192-94 ° C |
| 2-sulfanilamido-5 (or 8)-methoxy quinoxaline | b | 105 ° C |
| *2-sulfanilamido-5-chloroquinoxaline | b | 213-15 ° C |

In the preceeding list of compounds a refers to the lower melting isomer and b to the higher melting isomer.

In the preceding list, furthermore, the asterisk with which the 2-sulfanilamido-5-chloroquinoxaline was marginally marked, indicates, that the structure of this compound was determined absolutely by X-ray spectroscopy.

The pharmaceutical compositions prepared through the inventive use of the pharmaceutically active ingredients, are administered to animals suffering from a neoplatic disease state in such dosages, that the

corresponding animal receives an effective amount of one or more of the active ingredients of formula I. Usually the corresponding pharmaceutical compositions contain the active ingredient of formula I together with pharmaceutically acceptable diluents, adjuvants and/or carriers.

Unit dosage forms of the corresponding pharmaceutical compositions usually contain 0,1 to 500 mg of the active ingredient of formula I. The pharmaceutical compositions can be administered to provide a daily dosage of the active ingredient of formula I in the range of from 10 to 500 mg/kg of body weight of the animal to be treated. Parenteral administration and specially intraperitoneal administration is the preferred route for administering the corresponding pharmaceutical compositions.

The following examples are for illustrative purposes

EXAMPLE 1

## In Vitro Activity of 2-Sulfanilamido-5-chloro-quinoxaline

Tests carried out in accordance with the procedures described in Cancer Research 45, 2145-53, May 1985, Application of a Human Tumor Colony-forming Assay to New Drug Screening - Shoemaker et al.

| Colony Forming Assay Response Rates For 9 Tumor Types | | | |
|---|---|---|---|
| Tumor Type | Dose meq/ml | | |
| | 10 | 1 | 0.1 |
| Breast | 2/6 (33.3%) | 1/4 (25%) | 0/4 (0%) |
| Colorectal | 2/10 (20%) | 2/10 (0%) | 0/10 (0%) |
| Kidney | 3/10 (30%) | 1/4 (11.1%) | 0/0 (0%) |
| Lung | 5/11 (45%) | 3/8 (38%) | 1/8 (13%) |
| Melanoma | 4/13 (30.8%) | 2/8 (25%) | 0/8 (0%) |
| Ovary | 6/16 (37.5%) | 0/9 (0%) | 1/7 (14.3%) |
| Sarcoma | 1/1 (100%) | NT | NT |
| Skin | 0/1 (0%) | NT | NT |
| Uterine | 1/2 (50%) | NT | NT |
| Total for all tumors at 10 meq/ml - 24/70 (34.3%) | | | |
| Total for all tumors at 1 meq/ml - 7/48 (14.6%) | | | |
| Total for all tumors at 0.1 meq/ml - 2/47 (4.3%) | | | |
| Response = = 70% cell kill at = 10 meq/ml | | | |
| NT - Test faulted and experiment not included | | | |

EXAMPLE 2

## In Vivo Activity of 2-Sulfanilamido-5-chloro-quinoxaline

The test for antitumor activity in vivo using a new human tumor xenograft model, the LOX amelanotic melanoma in mice showed a significant increase in the life span of mice when treated with the title compound in doses of 449, 300 and 200 mg/kg/day.

**Claims**

**1.** Use of a compound of formula I

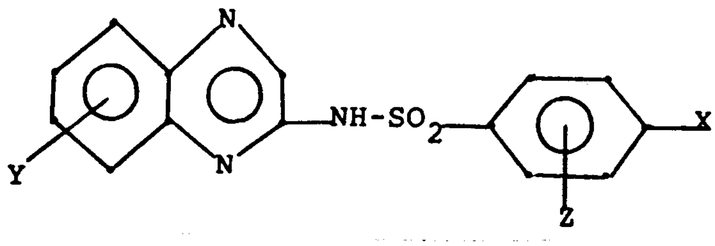

I

wherein:

Y is a substituent attached to any of the unsubstituted carbons on the quinoxaline ring which substituent is selected from $NH_2$, $NO_2$, $OCH_3$, hydroxy, hydrogen, methyl, fluoro, chloro, bromo or carboxy ;

Z is hydrogen or halo; and

X is selected from $NO_2$, acetylated amino groups, and groups having the formula:

$NH-(CH_2)_n-COOH$ or $NHCH_2SO_3H$

wherein

n is an integer of 1 - 6 and wherein furthermore in those compounds of formula I wherein at least one of the radicals Y and Z has another meaning than the hydrogen atom, the radical X can furthermore have the meaning of an amino group of formula $NH_2$,

as active ingredient for the preparation of pharmaceutical compositions for the treatment of a neoplastic disease.

**2.** Use according to claim 1 characterized in that the 2-sulfanilamido-5-chloro-quinoxaline is used for the preparation of the pharmaceutical composition. 3. Sulfanilamido-quinoxalines of formula Ia

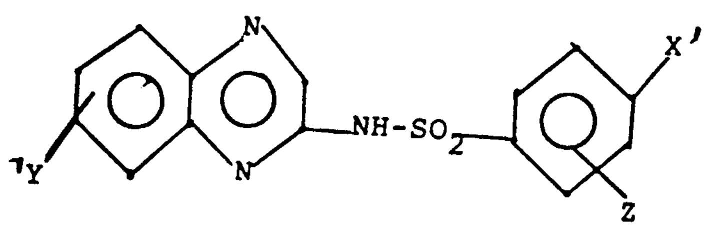

I a

wherein

Y' is a substituent attached to any of the unsubstituted carbons on the quinoxaline ring which substituent is selected from $NH_2$, $NO_2$, $OCH_3$, hydroxy, hydrogen, methyl, fluoro, chloro, bromo or carboxy

Z is hydrogen or halo; and

X' is selected from $NO_2$, $NH_2$, acetylated amino group and groups having the formula

$NH-(CH_2)_n-COOH$ or $NHCH_2SO_3H$

wherein

n is an integer of 1 - 6

with the provision that X' must not be a free or acetylated amino group if Z is hydrogen and furthermore Y' is $NH_2$, $NO_2$, $OCH_3$, hydrogen, methyl, fluoro, chloro, bromo or carboxy.

**Patentansprüche**

**1.** Verwendung einer Verbindung der Formel I

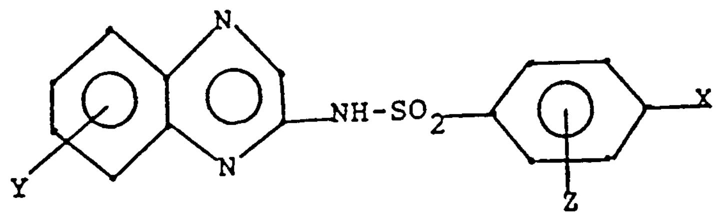

I

worin

Y ein Substituent ist, der an eines der unsubstituierten Kohlenstoffatome des Chinoxalinringes gebunden ist, wobei der Substituent ausgewählt ist aus $NH_2$, $NO_2$, $OCH_3$, Hydroxy, Wasserstoff, Methyl, Fluor, Chlor, Brom oder Carboxy,

Z Wasserstoff oder Halogen ist, und

X ausgewählt ist aus $NO_2$, acetylierten Aminogruppen und Gruppen der Formeln:

$NH-(CH_2)_n-COOH$ oder $NHCH_2SO_3H$,

worin

n eine ganze Zahl von 1 bis 6 ist und worin weiter in diesen Verbindungen der Formel I, in denen mindestens einer der Reste Y und Z eine andere Bedeutung als das Wasserstoffatom hat, der Rest X weiter die Bedeutung einer Aminogruppe der Formel $NH_2$ haben kann,

als aktiver Bestandteil für die Herstellung pharmazeutischer Zusammensetzungen für die Behandlung einer neoplastischen Erkrankung.

**2.** Verwendung nach Anspruch 1, dadurch gekennzeichnet, dass das 2-Sulfanilamido-5-chlor-chinoxalin für die Herstellung der pharmazeutischen Zusammensetzung verwendet wird.

**3.** Sulfanilamido-chinoxaline der Formel Ia

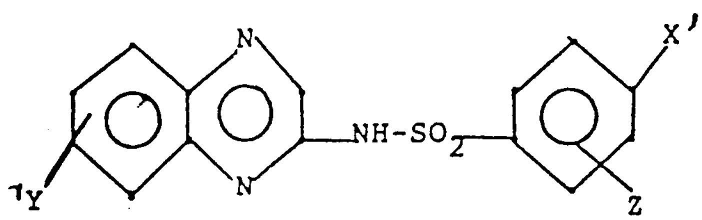

I a

worin

Y' ein Substituent ist, der an eines der unsubstituierten Kohlenstoffatome des Chinoxalinringes gebunden ist, wobei der Substituent ausgewählt ist aus $NH_2$, $NO_2$, $OCH_3$, Hydroxy, Wasserstoff, Methyl, Fluor, Chlor, Brom oder Carboxy,

Z Wasserstoff oder Halogen ist, und

X' ausgewählt ist aus $NO_2$, $NH_2$, einer acetylierten Aminogruppe und Gruppen der Formel

$NH-(CH_2)_n-COOH$ oder $NHCH_2SO_3H$,

worin

n eine ganze Zahl von 1 bis 6 ist

mit der Massgabe, dass X' keine freie oder acetylierte Aminogruppe sein darf, wenn Z Wasserstoff ist und weiterhin Y' $NH_2$, $NO_2$, $OCH_3$, Wasserstoff, Methyl, Fluor, Chlor, Brom oder Carboxy ist.

## Revendications

**1.** Utilisation d'un composé de formule I

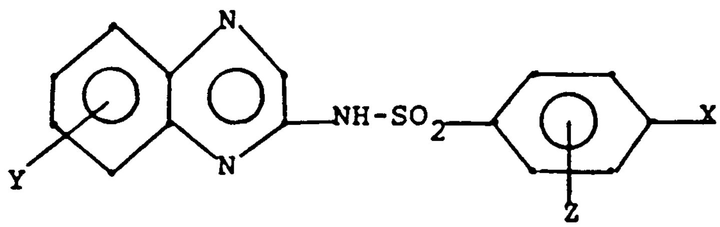

I

dans laquelle

Y est un substituant lié à n'importe lequel des carbones non-substitués du cycle quinoxaline lequel substituant est choisi parmi $NH_2$, $NO_2$, $OCH_3$, hydroxy, hydrogène, méthyle, fluoro, chloro, bromo ou carboxy ;

Z est de l'hydrogène ou un halo ; et

X est choisi entre $NO_2$, des groupements amino acétylés et des groupements ayant la formule :

$NH-(CH_2)_n-COOH$ ou $NHCH_2SO_3H$

dans laquelle n est un entier entre 1 et 6 et

dans laquelle de plus dans les composés de formule I dans lesquels au moins l'un des radicaux Y ou Z a une autre signification que l'atome d'hydrogène, le radical X peut de plus avoir comme signification un groupement amino de formule $NH_2$,

comme ingrédient actif pour la préparation de compositions pharmaceutiques pour le traitement d'une maladie néoplastique.

2. Utilisation selon la revendication 1 caractérisée en ce que la 2-sulfanilamido-5-chloro-quinoxaline est utilisée pour la préparation de la composition pharmaceutique.

3. Sulfanilamido-quinoxalines de formule Ia

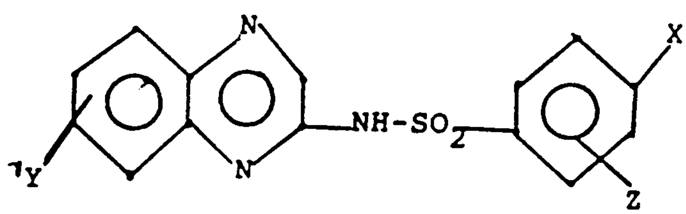

I a

Y' est un substituant lié à n'importe lequel des carbones non-substitués du cycle quinoxaline lequel substituant est choisi parmi $NH_2$, $NO_2$, $OCH_3$, hydroxy, hydrogène, méthyle, fluoro, chloro, bromo ou carboxy ;

Z est de l'hydrogène ou un halo ; et

X' est choisi entre $NO_2$, $NH_2$, des groupements amino acétylés et des groupements ayant la formule :

$NH-(CH_2)_n-COOH$ ou $NHCH_2SO_3H$

dans laquelle

n est un entier entre 1 et 6 et

avec la condition que X' ne doit pas être un groupement amino libre ou acétylé si Z est l'hydrogène et de plus Y' est un groupement $NH_2$, $NO_2$, $OCH_3$, hydrogène, méthyle, fluoro, chloro, bromo ou carboxy.